Europäisches Patentamt

European Patent Office

Office européen des brevets

(11) Publication number: **0 406 628 B1**

# EUROPEAN PATENT SPECIFICATION

(45) Date of publication of patent specification: **23.11.94**

(51) Int. Cl.5: **C12P 21/08**, C12N 5/12,
G01N 33/577, G01N 33/74

(21) Application number: **90111769.7**

(22) Date of filing: **21.06.90**

(54) **Monoclonal antibody against endothelin, hybridoma capable of producing the same and radioimmunoassay for endothelin by using the same.**

(30) Priority: **22.06.89 JP 160242/89**

(43) Date of publication of application:
**09.01.91 Bulletin 91/02**

(45) Publication of the grant of the patent:
**23.11.94 Bulletin 94/47**

(84) Designated Contracting States:
**AT BE CH DE DK ES FR GB GR IT LI LU NL SE**

(56) References cited:

**BIOCHEMICAL & BIOPHYSICAL RESEARCH COMMUN., vol. 161, no. 1, 30 May 1989, New York, NY (US); Y. SAITO et al., pp. 320-326&NUM;**

(73) Proprietor: **SHIONOGI & CO., LTD.**
**1-8, Doshomachi 3-chome,**
**Chuo-ku**
**Osaka 541 (JP)**

(72) Inventor: **Imura, Hiroo**
**59-2, Ichijojikitaomaru-cho**
**Sakyo-ku, Kyoto-shi, Kyoto-fu (JP)**
Inventor: **Nakao, Kazuwa**
**1-2, Oekitakutsukake-cho,**
**4-chome**
**Nishikyo-ku, Kyoto-shi, Kyoto-fu (JP)**

(74) Representative: **VOSSIUS & PARTNER**
**Postfach 86 07 67**
**D-81634 München (DE)**

## Description

The present invention relates to a monoclonal antibody against endothelin, a hybridoma capable of producing said antibody and a radioimmunoassay (hereinafter also referred to as "RIA") for endothelin by using the same.

Endothelin (hereinafter also referred to as "ET") is a 21-amino acid peptide purified from culture medium of porcine aortic endothelial cells (Yanagisawa, M., Kurihara, H., Kimura, S., Tomobe, Y., Kobayashi, M., Mitsui, Y., Yazaki, Y., Goto, K. and Masaki, T. (1988) Nature 332, 411-415). Preproendothelin mRNA is expressed not only in cultured endothelial cells but also in vivo in porcine aortic intima (Yanagisawa, M., Kurihara, H., Kimura, S., Tomobe, Y., Kobayashi, M., Mitsui, Y., Yazaki, Y., Goto, K., and Masaki, T. (1988) Nature 332, 411-415). The intravenous administration of ET induces the sustained elevation of arterial pressure with initial hypotension in anesthetized experimental animals such as rats (Yanagisawa, M., Kurihara, H., Kimura, S., Tomobe, Y., Kobayashi, M., Mitsui, Y., Yazaki, Y., Goto, K., and Masaki, T. (1988) Nature 332, 411-415; Yanagisawa, M., Inoue, A., Ishikawa, T., Kasuya, Y., Kimura, S., Kumagaye, A., Nakajima, K., Watanabe, T.X., Sakakibara, S., Goto, K. and Masaki, T. (1988) Proc. Natl. Acad. Sci. USA 85, 6964-6967; Miller, W.L., Redfield, M.M. and Burnett, Jr. J.C. (1989) J. Clin. Invest. 83, 317-320 and Firth JD, Ratcliffe, Raine AEG, Ledingham JGG: Endothelin: An important factor in acute renal failure? Lancet 1988;8621:1179-1181). It has been also reported that ET produces a vasoconstriction in isolated aortic, renal and coronary arteries in vitro (Yanagisawa, M., Kurihara, H., Kimura, S., Tomobe, Y., Kobayashi, M., Mitsui, Y., Yazaki, Y., Goto, K. and Masaki, T. (1988) Nature 332, 411-415; Yanagisawa, M., Inoue, A., Ishikawa, T., Kasuya, Y., Kimura, S., Kumagaye, A., Nakajima, K., Watanabe, T.X., Sakakibara, S., Goto, K. and Masaki, T. (1988) Proc. Natl. Acad. Sci. USA 85, 6964-6967 and Vanhoutte PM, Katusic ZS: Endothelium-derived contracting factor: endothelin and/or superoxide anion? TIPS 1989;9:229-230). It has been also reported that endothelin is a potent coronary vasoconstrictor in dogs in vivo (Kurihara, H., Yanagisawa, M., Yoshizumi, M., Kimura, S., Goto, K., Takaku, F., Masaki, T. and Yazaki, Y. (1988) 12th Scientific Meeting of the International Society of Hypertension, Kyoto, Japan, Abstract #0614 and Miller, W.L., Redfield, M. M. and Burnett, Jr. J.C. (1989) J. Clin. Invest. 83, 317-320).

These observations suggest that endothelin is involved in the control of cardiovascular homeostasis. Also, these observations raise the possibility that ET plays important roles in the development and/or maintenance of hypertension.

Recently, Yanagisawa et al. reported the presence of endothelin-related genes in the human genome, and products were designated ET-1, ET-2 and ET-3 (Inoue, A., Yanagisawa, M., Kimura, S., Kasuya, Y., Miyauchi, T., Goto, K. and Masaki, T. (1989) Proc. Natl. Acad. Sci. USA 86, 2863-2867). ET-1 is the original form isolated from culture medium of porcine aortic endothelial cells (Itoh, Y., Yanagisawa, M., Ohkubo, S., Kimura, C., Kosaka, T., Inoue, A., Ishida, N., Mitsui, Y., Onda, H., Fujino, M. and Masaki, T. (1988) FEBS Lett. 231, 440-444) and ET-3 is the sequence that was originally found in the rat genome (Yanagisawa, M., Inoue, A., Ishikawa, T., Kasuya, Y., Kimura, S., Kumagaye, A., Nakajima, K., Watanabe, T.X., Sakakibara, S., Goto, K. and Masaki, T. (1988) Proc. Natl. Acad. Sci. USA 85. 6964-6967). In addition, synthetic ET-2 also possesses strong vasoconstrictor and pressor actions.

It has been desired to provide immunoassays (RIAs) for endothelin with high precision which are suitable for investigating the implication of ET in hypertension and angiospasm and for diagnoses of diseases related to hypertension and angiospasm.

It is an object of the invention to provide a monoclonal antibody against endothelin having the association constant Ka of not less than $1 \times 10^{10} M^{-1}$, preferably not less than $5 \times 10^{10} M^{-1}$, more preferably not less than $7 \times 10^{10} M^{-1}$.

A further object of the invention is to provide a hybridoma capable of producing a monoclonal antibody against endothelin having an association constant Ka of not less than $1 \times 10^{10} M^{-1}$, preferably not less than $5 \times 10^{10} M^{-1}$, more preferably not less than $7 \times 10^{10} M^{-1}$.

It is a further object of the invention to provide a radioimmunoassay for endothelin, which is characterized by using a monoclonal antibody against endothelin having an association constant Ka of not less than $1 \times 10^{10} M^{-1}$, preferably not less than $5 \times 10^{10} M^{-1}$, more preferably not less than $7 \times 10^{10} M^{-1}$.

In accordance with the present invention, there is provided a monoclonal antibody against endothelin having an association constant Ka of not less than $1 \times 10^{10} M^{-1}$, a hybridoma capable of producing a monoclonal antibody against endothelin having an association constant Ka of not less than $1 \times 10^{10} M^{-1}$ and a radioimmunoassay for endothelin, which is characterized by using the monoclonal antibody against endothelin having an association constant Ka of not less than $1 \times 10^{10} M^{-1}$.

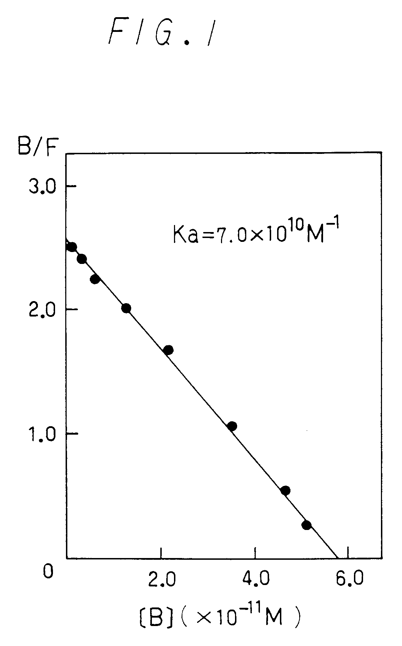

Fig. 1 is a graph showing a Scatchard plot of the binding of $^{125}$I-ET-1 to the monoclonal antibody KY-ET-1-I. Diluted ascitic fluid ($3 \times 10^6$ : 1) containing the monoclonal antibody KY-ET-1-I was incubated with

increasing concentrations of $^{125}$I-ET-1 (1.3 pM-54 pM) for 48 hr at 4°C, and the specific binding was determined after separation by the dextran-coated charcoal method.

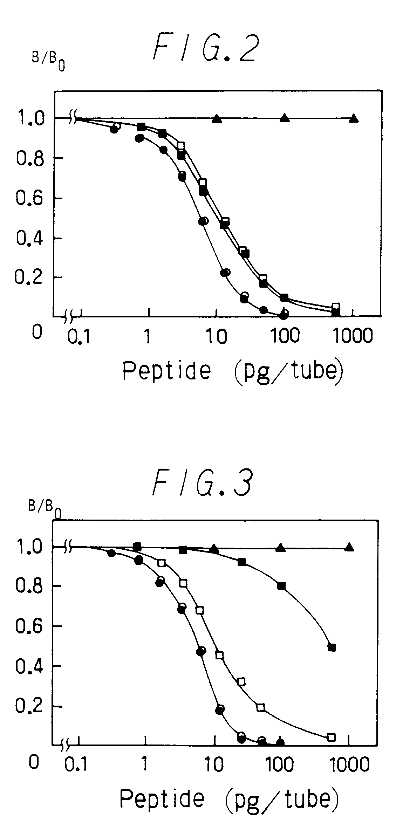

Fig. 2 shows a typical standard curve of ET-1 and cross-reactivities with other related peptides in the RIA with the monoclonal antibody KY-ET-1-I. Fig. 3 shows a typical standard curve of ET-1 and cross-reactivities with other related peptides in the RIA with ET-F5. In Figs. 2 and 3, ET-1 (closed circle), ET-2 (open circle), ET-3 (closed square), human big ET (open square), and ET[17-21] (closed triangle) were studied.

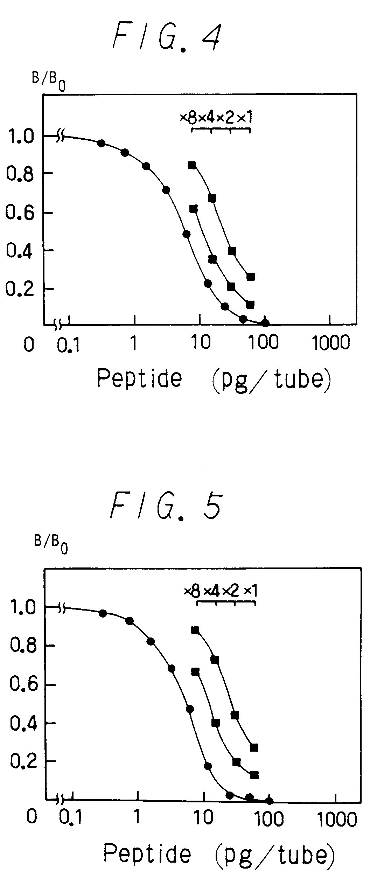

Fig. 4 shows a standard curve and a serial dilution curve of the extract from human plasma in the RIA with the monoclonal antibody KY-ET-1-I. Fig. 5 shows a standard curve and a serial dilution curve of the extract from human plasma in the RIA with ET-F5. In Figs. 4 and 5, standard curves (closed circle) and serial dilution curves (closed square) were studied.

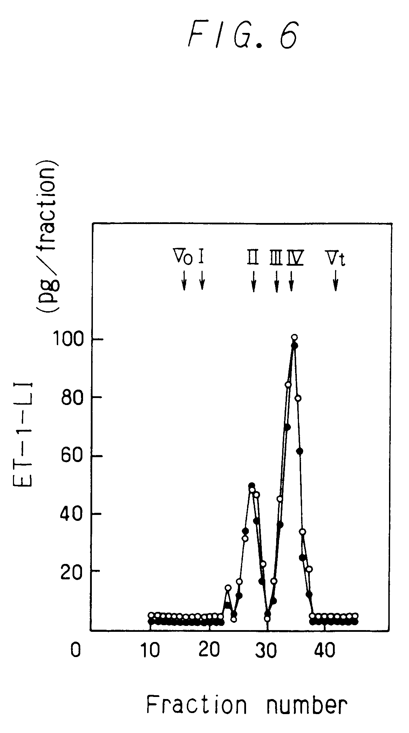

Fig. 6 shows a GPC profile of culture medium of bovine aortic endothelial cells applied to a Sephadex® G-50 column (0.7 X 50 cm). Arrows I, II, III and IV denote the elution positions of γ-human atrial natriuretic peptide (ANP) (13K), human big ET (4K), α-human ANP (3K) and ET-1 (2.5K), respectively. In Fig. 6, closed and open circles indicate immunoreactivities determined by the RIA with Monoclonal antibody KY-ET-1-I and by the RIA with ET-F5, respectively.

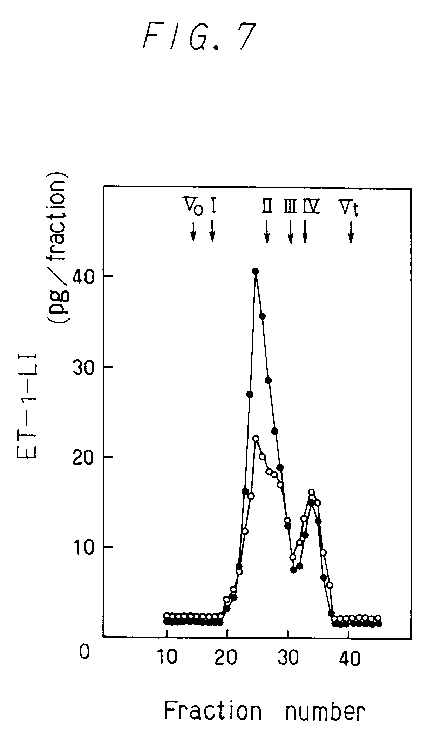

Fig. 7 shows a representative GPC profile of the human plasma extract applied to a Sephadex® G-50 column (0.7 X 50 cm). In Fig. 7, arrows and circles are the same as those in Fig. 6.

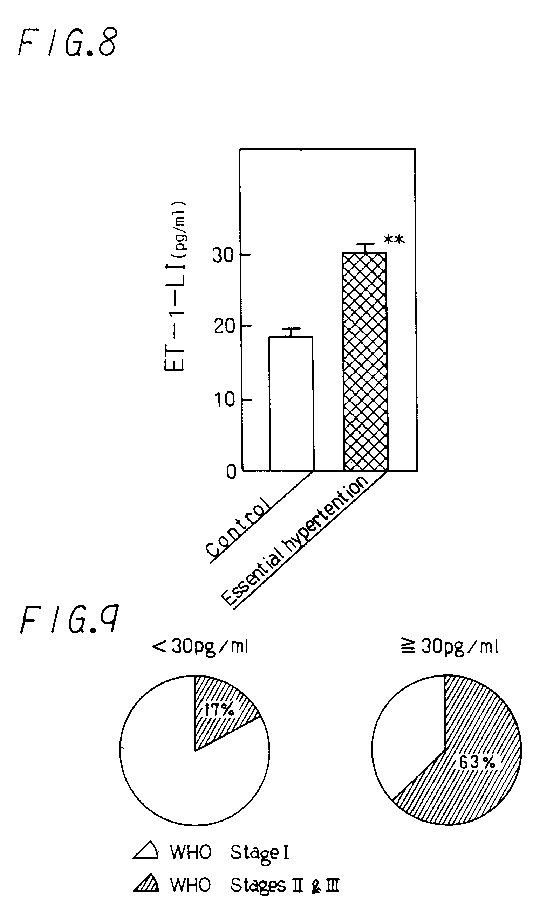

Fig. 8 is a graph showing the plasma ET-1-LI level in patients with essential hypertension in comparison with that in age-matched normal subjects.

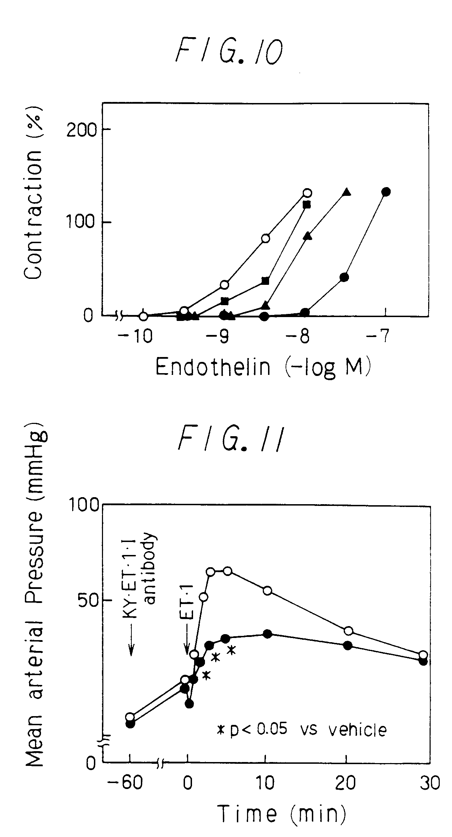

Fig. 9 is a graph showing a relationship between plasma endothelin level and incidence of organ complications.

Fig. 10 shows a dose-response curve of vasoconstriction against endothelin in isolated rat aortic strip. In Fig. 10, doses of monoclonal antibody KY-ET-1-I, of 5μg/mℓ (closed circle), 0.5 μg/mℓ (closed triangle), 0.05 μg/mℓ (closed square) and no monoclonal antibody, i.e. only vehicle (open circle) are shown.

Fig. 11 is a graph showing a relationship between time and the effect of the monoclonal antibody against elevation in arterial pressure induced by ET-1 in pithed rats. In Fig. 11, pretreatment of KY-ET-1-I (400 μg/kg) is shown as closed circle and only vehicle is shown as open circle. In Fig. 11, the arrows show the time when KY-ET-1-I (only closed circle) or ET-1 was administered, and the mark * shows a significant difference with $p < 0.05$ vs vehicle.

For the preparation of the monoclonal antibody of the present invention, methods can be used which have been put into operation to prepare monoclonal antibodies hitherto. That is, a monoclonal antibody can be produced by means of a known method from a hybridoma which is obtained by preparing hybridomas by cell fusion between an antibody-producing cell (for instance, a mouse spleen cell immunized with endothelin) and a myeloma cell and selecting a clone producing a desired antibody from the obtained hybridomas.

Hereinafter in the Examples of the present invention, the use of peptides as antigens, immunization thereby, preparation of monoclonal antibodies, characterization of the monoclonal antibodies, a radioimmunoassay with said monoclonal antibodies, culture of bovine aortic endothelial cells used as samples, subjects and blood sampling, separation and extraction of endothelin from plasma, measurement of the plasma ET-1-LI level, gel permeation chromatography, measurements of other plasma hormone levels, neutralization experiments and statistical analysis are explained. Hereinafter all per cents and parts are by weight unless otherwise noted.

Peptide

ET-1, ET-2 and ET-3 were purchased from Peptide Institute Inc. (Minoh, Japan). Human big ET (human prepro-ET[53-90]) and a common carboxy terminal fragment of ET (ET[17-21]) were synthesized by the solid phase method (Saito, Y., Nakao, K., Itoh, H., Yamada, T., Mukoyama, M., Arai, H., Hosoda, K., Shirakami, G., Suga, S., Jougasaki, M., Morichika, S., Imura, H.: Endothelin in human plasma and culture medium of aortic endothelial cells __ Detection and characterization with radioimmunoassay using monoclonal antibody. Biochem. Biophys. Res. Commun. 1989;161:320-326).

The purity of the peptides was confirmed by reversed phase high performance liquid chromatography and amino acid analysis.

Immunization

Synthetic ET-1 was conjugated to bovine thyroglobulin using the carbodiimide coupling procedure (Yoshimasa, T., Nakao, K., Ohtsuki, H., Li, S. and Imura, H. (1982) J. Clin. Invest. 69, 643-650 and Nakao, K., Sugawara, A., Morii, N., Sakamoto, M., Suda, M., Soneda, J., Ban, T., Kihara, M., Yamori, Y., Shimokura, M., Kiso, Y. and Imura, H. (1984) Biochem. Biophys. Res. Commun. 124, 815-821).

(1) Preparation of the hybridoma KY-ET-1-I

Adult female BALB/c mice were initially immunized by combined subcutaneous and intraperitoneal injections with the conjugate containing 10 $\mu$g of ET-1 emulsified in complete Freund's adjuvant (Mukoyama, M., Nakao, K., Sugawa, H., Morii, N., Sugawara, A., Yamada, T., Itoh, H., Shiono, S., Saito, Y., Arai, H., Mori, T., Yamada, H., Sano, Y., Imura, H.: A monoclonal antibody to $\alpha$-human atrial natriuretic polypeptide. Hypertension 1988;12:117-121; Mukoyama, M., Nakao, K., Yamada, T., Itoh, H., Sugawara, A., Saito, Y., Arai, H., Hosoda, K., Shirakami, G., Morii, N., Shiono, S., Imura, H.: A monoclonal antibody against N-terminus of $\alpha$-atrial natriuretic polypeptide ($\alpha$-ANP): a useful tool for preferential detection of naturally circulating ANP. Biochem. Biophys. Res. Commun. 1988;151:1277-1284 and Saito, Y., Nakao, K., Itoh, H., Yamada, T., Mukoyama, M., Arai, H., Hosoda, K., Shirakami, G., Suga, S., Jougasaki, M., Morichika, S., Imura, H.: Endothelin in human plasma and culture medium of aortic endothelial cells __ Detection and characterization with radioimmunoassay using monoclonal antibody. Biochem. Biophys. Res. Commun. 1989;161:320-326). They were boosted every 3 weeks and bled to test for the presence of antibody by RIA with $^{125}$I-ET-1 (Amersham IM 223, Buckinghamshire, England) 10-14 days after each booster injection. Three out of 10 mice gave a positive antibody response (ET-F5, ET-F9 and ET-F10).

(2) Preparation of the hybridomas KY-ET-1-II to -VI

The same treatment as described in (1) was carried out, except that 20 mice were used instead of 10 mice.

Five out of 20 mice gave a positive antibody response.

Preparation of monoclonal antibodies

Mice were further boosted intravenously with the conjugate containing 10 $\mu$g of the antigen 3 weeks after the last subcutaneous injection. Three days later the spleens were harvested from them for cell fusion. Fusion of spleen cells with a non-producing mouse myeloma cell line, X63-Ag8.653, was carried out in the ratio of 6:1 with 50 % polyethylene glycol (PEG 4000; Merck, Darmstadt, West Germany) according to the method described previously (Mukoyama, M., Nakao, K., Sugawa, H., Morii, N., Sugawara, A., Yamada, T., Itoh, H., Shiono, S., Saito, Y., Arai, H., Mori, T., Yamada, H., Sano, Y. and Imura, H. (1988) Hypertension 12, 117-121 and Mukoyama, M., Nakao, K., Yamada, T., Itoh, H., Sugawara, A., Saito, Y., Arai, H., Hosoda, K., Shirakami, G., Morii, N., Shiono, S. and Imura, H. (1988) Biochem. Biophys. Res. Commun. 151, 1277-1284). After the fusion, hybridomas were selected in hypoxanthine-aminopterin-thymidine medium containing 15 % fetal calf serum. Cells with high antibody titer were cloned twice by the limiting dilution technique. Positive clones were injected intraperitoneally into BALB/c mice to produce ascitic fluid with high concentration of antibody.

As a myeloma cell used in cell fusion a cell derived from mice, rats, humans or other amimals can be used. Among the obtained hybridomas, hybridoma KY-ET-1-I, hybridoma KY-ET-1-II, hybridoma KY-ET-1-III, hybridoma KY-ET-1-IV and hybridoma KY-ET-1-V have been deposited with the Fermentation Research Institute (FRI) under the accession numbers FERM BP-2365, FERM BP-2901, FERM BP-2902, FERM BP-2903 and FERM BP-2904, respectively. The deposition date of hybridoma KY-ET-1-I is March 30, 1989. The deposition dates of hybridomas KY-ET-1-II, hybridoma KY-ET-1-III, hybridoma KY-ET-1-IV and hybridoma KY-ET-1-V is May 15, 1990.

The present invention includes cell lines having the characteristics of the hybridomas of the present invention which produce a monoclonal antibody against endothelin having an association constant Ka of not less than 1 X $10^{10}$ M$^{-1}$, preferably not less than 5 X $10^{10}$ M$^{-1}$, more preferably not less than 7 X $10^{10}$ M$^{-1}$, and cell lines derived therefrom as well as hybridoma KY-ET-1-I, hybridoma KY-ET-1-II, hybridoma KY-ET-1-III, hybridoma KY-ET-1-IV, hybridoma KY-ET-1-V and hybridoma KY-ET-1-VI. Also the present invention includes monoclonal antibodies produced by the cell lines having the characteristics of the above hybridomas of the present invention and cell lines derived therefrom as well as monoclonal antibody KY-ET-1-I, monoclonal antibody KY-ET-1-II, monoclonal antibody KY-ET-1-III, monoclonal antibody KY-ET-1-IV, monoclonal antibody KY-ET-1-V and monoclonal antibody KY-ET-1-VI which are monoclonal antibodies against endothelin and are produced by hybridoma KY-ET-1-I, hybridoma KY-ET-1-II, hybridoma KY-ET-1-III, hybridoma KY-ET-1-IV, hybridoma KY-ET-1-V and hybridoma KY-ET-1-VI, respectively.

## Characterization of the monoclonal antibody

Isotyping of the monoclonal antibody was performed by the Ouchterlony technique (Mouse Monoclonal Typing Kit; Miles Laboratories, Inc., Elkhart, IN) (Mukoyama, M., Nakao, K., Yamada, T., Itoh, H., Sugawara, A., Saito, Y., Arai, H., Hosoda, K., Shirakami, G., Morii, N., Shiono, S., Imura, H.: A monoclonal antibody against N-terminus of $\alpha$-atrial natriuretic polypeptide ($\alpha$-ANP): a useful tool for preferential detection of naturally circulating ANP. Biochem. Biophys. Res. Commun. 1988;151:1277-1284 and Saito, Y., Nakao, K., Itoh, H., Yamada, T., Mukoyama, M., Arai, H., Hosoda, K., Shirakami, G., Suga, S., Jougasaki, M., Morichika, S., Imura, H.: Endothelin in human plasma and culture medium of aortic endothelial cells __ Detection and characterization with radioimmunoassay using monoclonal antibody. Biochem. Biophys. Res. Commun. 1989;161:320-326). Binding affinity was analyzed by constructing a Scatchard plot of ET-1 with the RIA described below. The monoclonal antibody of the present invention is suitable for a competitive immunoassay and has an association constant Ka of not less than $1 \times 10^{10}$ $M^{-1}$. The association constant Ka is preferably not less than $5 \times 10^{10} M^{-1}$, more preferably not less than $7 \times 10^{10} M^{-1}$. The association constant Ka is much more preferably not less than $1 \times 10^{11} M^{-1}$.

## Radioimmunoassay (RIA)

The assay buffer of the RIA was 0.1 M phosphate buffer, pH 7.0, containing 0.5 % gelatin (Merck), 1 mM $Na_2$EDTA, 0.2 mM cystine, 0.1 % Triton X-100 and 0.01 % merthiolate and was used to dissolve all reagents. The RIA incubation mixture consisted of 50 $\mu l$ of standard ET-1 or sample, 50 $\mu l$ of antibody (ascites or antisera) and 100 $\mu l$ of assay buffer. After the mixture was incubated for 24 hr at 4°C, $^{125}$I-ET-1 (approximately 3,000 cpm) was added and the mixture was incubated for another 24-hour period. Bound and free ligands were separated by adding 250 $\mu l$ of assay buffer and 1 ml of a suspension of dextran-coated charcoal consisting of 400 mg of Norit SX Plus (N.V. Norit-Vereeninging, Holland) and 40 mg of Dextran T-70 (Pharmacia Fine Chemicals, Uppsala, Sweden) in 0.05 M phosphate buffer (pH 7.4) containing 0.01 % merthiolate. Hereinafter, using the above-mentioned RIAs, it is explained that the concentration and the molecular form of a substance having ET-1-like immunoreactivity (hereinafter referred to as "ET-1-LI") were investigated in culture medium of bovine aortic endothelial cells and human plasma.

## Culture of bovine aortic endothelial cells

Bovine aortic endothelial cells were dispersed with collagenase, as previously reported (Hagiwara, H., Shimonaka, M., Morisaki, M., Ikekawa, N. and Inada, Y. (1984) Thrombos. Res. 33, 363-379). Dispersed endothelial cells were cultured in Dulbecco's modified Eagle medium with 15 % fetal calf serum. The culture medium was changed to the serum-free medium (GIT, Nihon Pharmaceutical Co. Ltd., Tokyo, Japan) 2 hr before the sampling.

## Subjects and blood sampling

(1) ET-1-LI level in human plasma of normal subjects

Eight normal male volunteers, aged 26-40 years old, were studied. Blood samplings for the measurement of the plasma ET-1-LI level were performed from the antecubital vein at a recumbent position after an overnight fast.

(2) Plasma ET-1-LI level in patients with essential hypertension

The inventors studied 20 patients with essential hypertension before drug treatment (age, mean ± SD; 51.7 ± 2.8 years) and 12 age-matched control subjects (age; 50.0 ± 0.6 years). According to the World Health Organization classification, hypertensive patients consisted of 13 in stage I, 5 in stage II and 2 in stage III. All patients had a serum creatinine level of less than 1.5 mg/dl. Their systolic and diastolic blood pressure and heart rate were 160 ± 5 mmHg, 98 ± 2 mmHg and 71 ± 3 beats/min, respectively. Their daily uptake of sodium chloride was 10.6 ± 0.7 g. Blood was collected from an antecubital vein in the recumbent position after an overnight fast.

Separation and extraction of endothelin from plasma

Separation and extraction of ET from plasma was carried out using polystyrene beads coated with the purified monoclonal antibody (Monoclonal antibody KY-ET-1-I).

The monoclonal antibody was purified using a Protein A affinity column (Affi-Gel Protein A MAPS II Kit, Bio-Rad Laboratories, Richmond, CA). Polystyrene beads (diameter 6.5 mm, Ichiko, Komako, Japan) were immersed in 0.05 M phosphate buffer (pH 7.5) containing 0.01 % of the purified monoclonal antibody, 0.9 % of sodium chloride and 0.05 % of sodium azide for 24 hr at 4°C to prepare antibody-coated beads.

The extraction mixture consisting of 1 ml plasma and 1 ml phosphate buffer (0.1 M, pH 7.0) containing 0.1 % gelatin, 1 mM EDTA, 0.2 mM cystine and aprotinin (2,000 kallikrein inactivator units/ml Ohkura Pharmaceutical Co. Ltd., Kyoto, Japan), and the antibody-coated beads were incubated at 4°C for 24 hr with gentle shaking. The beads were washed with 0.05 M phosphate buffer and then, were heated in 300 $\mu$l of 0.1 M phosphate buffer (pH 7.0) containing 0.5 % gelatin at 85°C for 30 min using a heating block (Yamato, model HF-21, Tokyo, Japan). For the measurement of the plasma ET-1-LI level, the heated extract was directly used for the RIA as a sample.

Measurement of the plasma ET-1-LI level

The plasma ET-1-LI level was measured by a RIA with the monoclonal antibody (Monoclonal antibody KY-ET-1-I) as previously reported (Saito, Y., Nakao, K., Itoh, H., Yamada, T., Mukoyama, M., Arai, H., Hosoda, K., Shirakami, G., Suga, S., Jougasaki, M., Morichika, S., Imura, H.: Endothelin in human plasma and culture medium of aortic endothelial cells __ Detection and characterization with radioimmunoassay using monoclonal antibody. Biochem. Biophys. Res. Commun. 1989;161;320-326). Recoveries of 25 pg and 50 pg of ET-1 added to 1 ml of plasma were 52 % and 55 %, respectively (Saito, Y., Nakao, K., Itoh, H., Yamada, T., Mukoyama, M., Arai, H., Hosoda, K., Shirakami, G., Suga, S., Jougasaki, M., Morichika, S., Imura, H.: Endothelin in human plasma and culture medium of aortic endothelial cells __ Detection and characterization with radioimmunoassay using monoclonal antibody. Biochem. Biophys. Res. Commun. 1989;161;320-326). The recovery of 50 pg of big ET which had been added to 1 ml of plasma was 48%. Inter- and intra-assay variations in the RIA were 6.8 ± 2.8 % and 7.3 ± 2.2 %, respectively (Saito, Y., Nakao, K., Itoh, H., Yamada, T., Mukoyama, M., Arai, H., Hosoda, K., Shirakami, G., Suga, S., Jougasaki, M., Morichika, S., Imura, H.: Endothelin in human plasma and culture medium of aortic endothelial cells __ Detection and characterization with radioimmunoassay using monoclonal antibody. Biochem. Biophys. Res. Commun. 1989;161;320-326).

Gel permeation chromatography (GPC)

GPC was performed on a Sephadex® G-50 (Pharmacia Fine Chemicals, Uppsala, Sweden) column (0.7 X 50 cm). The column was eluted using the assay buffer as solvent (Nakao, K., Nakai, Y., Oki, S., Horii, K. and Imura, H. (1978) J. Clin. Invest. 62, 1395-1398). The flow rate was 3 ml/hr and the fraction volume was 0.5 ml. As molecular weight markers, blue dextran (for void volume; Vo), $\gamma$-human atrial natriuretic peptide (for a molecular weight of 13K), human big ET (for a molecular weight of 4K), $\alpha$-human atrial natriuretic peptide (for a molecular weight of 3K), ET-1, ET-2, ET-3, and $^{125}$I for (salt peak) were used. For the analysis of the culture medium, 200 $\mu$l of the medium were directly applied to the column. For the analysis of the plasma, the extract from 30-ml human plasma was used. ET was extracted using monoclonal antibody-coated beads, as mentioned above.

Measurements of other plasma hormone levels

The present inventors also measured plasma levels of various hormones, such as atrial natriuretic peptide, arginine vasopressin, norepinephrine, epinephrine and aldosterone, and plasma renin activity, as previously reported (Saito, Y., Nakao, K., Nishimura, K., Sugawara, A., Okumura, K., Obata, K., Sonoda, R., Ban, T., Yasue, H., Imura, H.: Clinical application of atrial natriuretic polypeptide to patients with congestive heart failure: Beneficial effects on left ventricular function. Circulation 1987;76;115-124; Saito, Y., Nakao, K., Sugawara, A., Nishimura, K., Sakamoto, M., Morii, N., Yamada, T., Itoh, H., Shiono, S., Kuriyama, T., Hirai, M., Ohi, M., Ban, T., Imura, H.: Atrial natriuretic polypeptide during exercise in healthy man. Acta

EP 0 406 628 B1

Endocrinologica 1987;116:59-65 and Yamada, T., Nakao, K., Morii, N., Itoh, H., Shiono, S., Sakamoto, M., Sugawara, A., Saito, Y., Ohno, H., Kanai, A., Katsuura, G., Eigyo, M., Matsushita, A., Imura, H.; Central effect of atrial natriuretic polypeptide on angiotensin II-stimulated vasopressin secretion in conscious rats. Eur. J. Pharmacol. 1986;125:453-456).

Neutralization experiments

Contractile responses of rat aortic strips were performed as previously reported (Toda, S., Nakajima, S., Miyazaki, M., Ueda, M.: Vasodilation induced by pinacidil in dogs __ comparison with hydralizine and nifedipine. J. Cardiovasc. Pharmacol. 1985;7:1118-1126). Aortic strips were incubated in an organ bath perfused at 37°C with balanced salt solution containing 139.7 mEq $Na^+$, 5.4 mEq $K^+$, 2.2 mEq $Ca^{2+}$, 1.0 mEq $Mg^{2+}$, 131.5 mEq $Cl^-$ and 20 mEq $HCO_3^-$. Purified monoclonal antibody (0.05 $\mu$g/ml - 5 $\mu$g/ml) was added 3 min before the ET-1 administration.

The effects of the monoclonal antibody of the present invention on ET-1-induced pressor action were examined in pithed rats, in which the evaluation of the vascular smooth muscle contraction or relaxation was directly assayed, because of the lack of the autonomic nervous reflex. Pithed rats were made as previously reported (Nakajima, S., Ueda, M.: Pharmacological studies on pinacidil, a new antihyperagent, 3 studies on the vasodilating effects in isolated monkey arteries and the peripheral hypotensive mechanism in SHR. Folia Pharmacol. Japon 1987;90:13-21). Arterial pressure was monitored directly via the catheter inserted into the femoral artery. Purified antibody or the same dose of mouse immunoglobulin $G_1$ (400 $\mu$g/kg) was injected 60 min before the bolus injection of ET-1 (0.3 nmol/kg).

Statistical analysis

Values are expressed as mean ± standard error of mean (SE). Univariate analysis of mean data between groups was performed by the Student's t test. Univariate analysis of proportions between groups was performed by the chi-square test. Linear regression analysis was used to determine correlations between results.

The present invention is more specifically described and explained in the following Examples.

Example 1

Preparation and characterization of the monoclonal antibody

As mentioned above, fusion of a mouse spleen cell immunized with ET-1 with a non-producing mouse myeloma cell was carried out. After the fusion, ET-antibody-producing cells were recognized in two of the wells. After further culture and cloning, one clone which produced an antibody with the highest titer was selected for expansion and characterization. The obtained monoclonal antibody (Monoclonal antibody KY-ET-1-I) belonged to the $IgG_1$ subclass as determined by the Ouchterlony technique. Analysis by a Scatchard plot revealed a high affinity for ET-1 with a Ka of 7.0 X $10^{10} M^{-1}$. The results are shown in Fig. 1.

Examples 2 to 6

In the same manner as described in Example 1, monoclonal antibody KY-ET-1-II, monoclonal antibody KY-ET-1-III, monoclonal antibody KY-ET-1-IV, monoclonal antibody KY-ET-1-V and monoclonal antibody KY-ET-1-VI were obtained. It was determined by the Ouchterlony technique that monoclonal antibody KY-ET-1-II, monoclonal antibody KY-ET-1-III, monoclonal antibody KY-ET-1-IV, monoclonal antibody KY-ET-1-V and monoclonal antibody KY-ET-1-VI are belonging to the subclasses $IgG_1$, $IgG_{2a}$, $IgG_1$, $IgG_1$ and $IgG_{2a}$, respectively. As the result of an assay by a Scatchard plot, the association constants Ka are 2.1 X $10^{11}$, 2.4 X $10^{11}$, 4.8 X $10^{11}$, 5.3 X $10^{10}$ and 6.0 X $10^{10} M^{-1}$. It was shown that the above monoclonal antibodies have strong affinity to ET-1. In Table 1, the characteristics of each monoclonal antibody to ET-1 are shown.

7

Table 1

| Antibody | IgG subclass | Ka ($M^{-1}$) | $IC_{50}$ (pg/tube) | Cross-reactivities (%) | | | |
|---|---|---|---|---|---|---|---|
| | | | | ET-2 | ET-3 | human big ET | ET[17-21] |
| KY-ET-1-I | $IgG_1$ | $7.0 \times 10^{10}$ | 6 | 100 | 60 | 100 | 0.03 |
| KY-ET-1-II | $IgG_1$ | $2.1 \times 10^{11}$ | 4 | 90 | 40 | 100 | <0.01 |
| KY-ET-1-III | $IgG_{2a}$ | $2.4 \times 10^{11}$ | 4 | 90 | 35 | 100 | <0.01 |
| KY-ET-1-IV | $IgG_1$ | $4.8 \times 10^{11}$ | 2 | 80 | 20 | 80 | <0.01 |
| KY-ET-1-V | $IgG_1$ | $5.3 \times 10^{10}$ | 10 | 90 | 15 | 50 | <0.01 |
| KY-ET-1-VI | $IgG_{2a}$ | $6.0 \times 10^{10}$ | 8 | 80 | 3 | 50 | <0.01 |

IgG = immunoglobulin G., $IC_{50}$ = value of 50 % inhibitory concentration,
Ka = association constant

Analysis by the Scatchard plot method revealed that all antibodies of the present invention have high affinities for ET-1 (Ka: $5.3 \times 10^{10} M^{-1}$ - $4.8 \times 10^{11}$ $M^{-1}$). Although all antibodies of the present invention recognized ET-2 (cross-reactivity; from 80 % to 100 %), cross-reactivities with ET-3 were from 3 % to 60 % on a molar basis. KY-ET-1-I exhibited the highest cross-reactivity with ET-3 among the 6 antibodies of the present invention. All antibodies of the present invention also recognized human big ET. As shown in Table 1, the value of 50 % inhibitory concentration of each RIA with each one of the 6 antibodies of the present

8

invention ranged from 2 pg/tube to 10 pg/tube. The RIA with monoclonal antibody KY-ET-1-IV was the most sensitive for ET-1.

Example 7

RIA for ET-1

Ascites (Monoclonal antibody KY-ET-1-I) and antiserum (ET-F5) were used as antibodies in RIAs at the final dilution of $3.0 \times 10^7 : 1$ and $1.5 \times 10^4 : 1$, respectively. The standard curves in RIAs using monoclonal antibody KY-ET-1-I and ET-F5 are shown in Figs. 2 and 3, respectively.

In the RIA with monoclonal antibody KY-ET-1-I, ET-1, ET-2 and ET-3 were detected. On the other hand, in the RIA with ET-F5, ET-3 was very weakly recognized.

In both RIAs, the value of 50 % inhibitory concentration was 6 pg/tube and the minimal detectable amount was 0.5 pg/tube. As shown in Table 1, the value of 50 % inhibitory concentration of each RIA with each one of the 6 antibodies of the invention ranged from 2 pg/tube to 10 pg/tube. The RIA with monoclonal antibody KY-ET-1-IV was the most sensitive one for ET-1. Although the RIA with ET-F5 recognized ET-3 very weakly (cross-reactivity: 2 %), the RIA with monoclonal antibody KY-ET-1-I shows cross-reactivity of 60 % with ET-3. Cross-reactivities with ET-2 and human big ET were 100 % on a molar basis in each RIA.

Although all antibodies of the invention recognized ET-2 (cross-reactivity; from 80 % to 100 %), cross-reactivities with ET-3 were from 3 % to 60 % on a molar basis. The monoclonal antibody KY-ET-1-I exhibited the highest cross-reactivity with ET-3 among the 6 antibodies of the present invention.

These RIAs did not recognize the common carboxy terminal fragment of ET in ET-1, ET-2 and ET-3, ET[17-21] (Fig.2 ).

Example 8

ET-1-LI in culture medium of bovine aortic endothelial cells

ET-1-LI was detected in the culture medium of bovine aortic endothelial cells in both RIAs with monoclonal antibody KY-ET-1-I and with ET-F5. The dilution curve of the medium was parallel to the standard curves in each RIA. The secretory rate of ET from endothelial cells was 0.45 ng/hr/$10^6$ cells during 2-5 hr after the beginning of serum-free culture. GPC in combination with the RIAs revealed that ET-1-LI in the culture medium consisted of two major and one minor components (Fig. 6). The two major peaks emerged at the elution positions of synthetic human big ET and ET-1, respectively. ET-1-LI in the two peaks determined by the RIA with monoclonal antibody KY-ET-1-I was identical to that determined by the RIA with ET-F5. The minor peak was eluted at the position of a molecule larger than big ET.

Example 9

ET-1-LI level in human plasma of normal subjects

ET-1-LI was detected in the extract of human plasma in both RIAs. Serial dilution curves of plasma extracts were parallel to the standard curve in each RIA (Figs. 4 and 5). Plasma ET-1-LI levels in normal healthy volunteers are shown in Table 2. The plasma ET-1-LI level was 19.1 ± 1.1 pg/ml in the RIA with monoclonal antibody KY-ET-1-I and 9.9 ± 0.5 pg/ml in the RIA with ET-F5.

EP 0 406 628 B1

Table 2

| Subject | Age (yr) | Sex | ET-1-LI (pg/ml) | |
|---|---|---|---|---|
| | | | KY-ET-1-I | ET-F5 |
| 1 K.N. | 40 | M | 24.4 | 12.2 |
| 2 H.I. | 31 | M | 21.4 | 10.4 |
| 3 Y.S. | 32 | M | 19.1 | 9.6 |
| 4 K.H. | 30 | M | 19.1 | 8.7 |
| 5 G.S. | 31 | M | 19.0 | 11.3 |
| 6 M.J. | 30 | M | 18.6 | 9.3 |
| 7 Y.I. | 31 | M | 14.6 | 8.7 |
| 8 O.N. | 26 | M | 17.0 | 9.4 |
| mean ± SE | 31.4 ± 1.5 | | 19.1 ± 1.1 | 9.9 ± 0.5 |
| SE = standard error | | | | |

Example 10

Plasma ET-1-LI level in patients with essential hypertension

The plasma ET-1-LI level in patients (n = 20) with essential hypertension was 30.1 ± 1.4 pg/ml and was significantly elevated compared with that in age-matched control subjects (n = 12) (18.5 ± 0.9 pg/ml) (p < 0.01) (Fig. 8). Patients in stages II and III showed higher plasma ET-1-LI level than patients in stage I (33.9 ± 1.8 pg/ml vs 28.1 ± 1.7 pg/ml). Patients were divided according to their plasma ET-1-LI levels into those with levels below (Group A) and above (Group B) the average value (30 pg/ml) of the total patients. Five out of 8 patients in Group B belonged to stage II or III, whereas 2 out of 12 patients in Group A were in stage II or III. Thus, the incidence of organ complications in Group B was significantly higher than that in Group A (Fig. 9).

The correlation coefficients between the plasma ET-1-LI level and clinical and laboratory parameters are summarized in Table 3.

10

Table 3

| | Parameter | Correlation coefficient |
|---|---|---|
| Hemodynamics | SBP | r = 0.11 |
| | DBP | r = -0.13 |
| | HR | r = 0.13 |
| Hormone | ANP | r = -0.08 |
| | PRA | r = -0.002 |
| | PAC | r = 0.40 |
| | NE | r = 0.41 |
| | E | r = 0.04 |
| | AVP | r = 0.19 |
| Renal function | CRE | r = -0.07 |
| | BUN | r = 0.29 |
| Age | | r = 0.24 |

SBP = systolic blood pressure, DBP = diastolic blood pressure, HR = heart rate, ANP = plasma atrial natriuretic peptide level, PRA = plasma renin activity, PAC = plasma aldosterone concentration, NE = plasma norepinephrine concentration, E = plasma epinephrine concentration, CRE = serum creatinine level, BUN = serum blood urea nitrogen level, AVP = plasma arginine vasopressin concentration.

EP 0 406 628 B1

There was no significant correlation between the plasma ET-1-LI level and systolic or diastolic blood pressure in patients with essential hypertension. The plasma ET-1-LI level was not correlated with any plasma hormone levels, which were involved in the control of blood pressure and water balance.

Example 11

Neutralization experiments

Synthetic ET-1 induced a vasoconstriction in isolated rat aortic strips in a dose-dependent manner. The administration of purified monoclonal antibody (Monoclonal antibody KY-ET-1-I) dose-dependently shifted the dose-response curve to the right (Fig. 10). In pithed rats, the elevation in the mean arterial pressure induced by ET-1 was significantly suppressed by the pretreatment with the purified monoclonal antibody of the invention 60 min before the ET-1 administration (Fig. 11).

Fig. 7 shows the representative GPC profile of ET-1-LI in the extract of human plasma. ET-1-LI in human plasma consisted of two major apparent components. The peak with the small molecular size was located at the elution position of synthetic ET-1. The other peak was eluted at the preceding fraction (6K) of the position of human big ET (4K). There existed ET-1-LI at the elution position of big ET. The ET-1-LI with the small molecule form corresponded to 25.8 ± 1.5 % (4.9 pg/ml) of ET-1-LI in the RIA with monoclonal antibody KY-ET-1-I but 36.7 ± 2.4 % (3.6 pg/ml) in the RIA with ET-F5. There was no significant difference in ET-1-LI of the small molecule form between the two RIAs, while the ET-1-LI in the large molecule form determined by the RIA with ET-F5 was about 70 % of that determined by the RIA with monoclonal antibody KY-ET-1-I.

The GPC profile of ET-1-LI in culture medium of bovine aortic endothelial cells revealed the presence of two peaks eluting at the positions of synthetic big ET and ET-1. The GPC profile in the RIA with monoclonal antibody KY-ET-1-I was almost identical with that in the RIA with ET-F5. These findings indicate that endothelial cells secrete big ET as well as the small molecular form of ET. Because of the low cross-reactivity with ET-3 in the RIA with ET-F5, endothelial cells produce little ET-3. In addition, since there is no evidence that the ET-2 gene is expressed in endothelial cells (Inoue, A., Yanagisawa, M., Kimura, S., Kasuya, Y., Miyauchi, T., Goto, K. and Masaki, T. (1989) Proc. Natl. Acad. Sci. USA 86, 2863-2867), the small form of ET in the culture medium is considered to be mainly ET-1. This observation on big ET and ET-1 in the culture medium of aortic endothelial cells is consistent with the hypothesis proposed previously (Yanagisawa, M., Kurihara, H., Kimura, S., Tomobe, Y., Kobayashi, M., Mitsui, Y., Yazaki, Y., Goto, K. and Masaki, T. (1988) Nature 332, 411-415). No difference in the immunoreactivity in each molecular form of ET determined by the two RIAs also indicates the validity of the RIAs of the present invention.

The present invention demonstrated the existence of ET-1-LI in human plasma with the mean values of 19.1 ± 1.1 pg/ml determined by the RIA with monoclonal antibody KY-ET-1-I and of 9.9 ± 0.5 pg/ml determined by the RIA with ET-F5. This observation raises the possibility that ET is a circulating hormone as well as a local hormone.

In the present invention,6 different monoclonal antibodies against ET-1 have been prepared and applied to RIAs and neutralization experiments. Association constants of these antibodies ranged from 5.3 X $10^{10}M^{-1}$ to 4.8 X $10^{11}M^{-1}$. All antibodies of the present invention have higher affinities than the ET-1 monoclonal antibody reported recently (Suzuki, N., Matsumoto, H., Kitada, C., Masaki, T., Fujino, M.: A sensitive sandwich-enzyme immunoassay for human endothelin. J. Immunol. Method 1989;118:245-250). The value of the 50 % inhibitory concentration in each RIA with each one of the 6 antibodies of the present invention ranged from 2 pg/tube to 10 pg/tube. Using the RIA with the most sensitive antibody (Monoclonal antibody KY-ET-1-IV), plasma ET-1-LI in human and rat plasma can be directly detected. In the present invention, monoclonal antibody-coated beads for the extraction of ET from plasma were used. The major advantage of using affinity beads for the extraction is the specificity in comparison with other extraction methods, such as Sep-Pak $C_{18}$ cartridge. Because the cross-reactivity with ET-3 was from 3 % to 60 % for these antibodies, each antibody recognizes different epitopes. Therefore, these antibodies can be used for the two-antibody sandwich immunoassay.

In the present invention,an elevated plasma ET-1-LI level in patients with essential hypertension is also demonstrated. This observation clearly indicates the possible pathophysiological significance of ET in essential hypertension. The plasma ET-1-LI level of patients in stages II and III were significantly higher than that of those in stage I. In addition, patients with higher plasma ET-1-LI level tended to have organ complications more frequently. These observations suggest the possibility that the elevation of the plasma ET-1-LI level is related to organ complications including atherosclerosis associated with hypertension.

Recently the present inventors reported that ET-1-LI in normal plasma consists of ET-2, big ET and another precursor form of ET (6K daltons), using gel permeation chromatography (Saito, Y., Nakao, K., Itoh, H., Yamada, T., Mukoyama, M., Arai, H., Hosoda, K., Shirakami, G., Suga, S., Jougasaki, M., Morichika, S., Imura, H.: Endothelin in human plasma and culture medium of aortic endothelial cells __ Detection and characterization with radioimmunoassay using monoclonal antibody. Biochem, Biophys. Res. Commun. 1989;161;320-326). It remains to be clarified which component(s) is(are) preferentially increased in the patients with essential hypertension. Parallel increases of ET-1 and big ET were also reported in patients with acute myocardial infarction (Miyauchi, T., Yanagisawa, M., Tomizawa, T., Sugishita, Y., Suzuki, N., Fujino, M., Ajisaka, R., Goto, K., Masaki, T.: Increased plasma concentrations of endothelin-1 and big endothelin-1 in acute myocardial infarction. Lancet 1989;ii:8653:53-54). It is, therefore, possible that the elevation of the plasma ET-1-LI level was due to parallel increases of ET-1, big ET and another component.

The role of the increased plasma ET-1-LI level in essential hypertension is not clear at present. Recently, Komuro et al. reported that ET-1 possesses a proliferative effect on vascular smooth muscle cells (Komuro, L., Kurihara, H., Sugiyama, T., Takaku, F., Yazaki, Y.: Endothelin stimulates c-fos and c-myc expression and proliferation of vascular smooth muscle cells. FEBS Lett. 1988;238:249-252). It may be possible, therefore, that the increased secretion of ET from endothelial cells plays an obligatory role in the development and aggravation of atherosclerotic changes inthe underlying intima and media and thereby is related to organ complications. On the other hand, the low dose of ET-1 is reported to cause the vaso-dilatation instead of vasoconstriction via the production of prostaglandin $I_2$, and/or endothelium-derived relaxant factor (de Nucci, G., Thomas, R., D'Orleans-Juste, P., Antunes, E., Walder, C., Warner, TD., Vane, JR.: Pressor effects of circulating endothelin are limited by its removal in the pulmonary circulation and by the release of prostacyclin and endothelium-derived relaxing factor. Proc. Natl. Acad. Sci. USA 1988;85:9797-9800; Pohl, U., Busse, R.: Differential vascular sensitivity to luminally and adventitially applied endothelin-1. J. Cardiovasc. Pharmacol. 1989;13[Suppl. 5]:S188-S190 and Minkes RK., MacMillan LA., Bellan, JA., Kerstein, MD., McNamra, DB., Kadowitz, PJ.: Analysis of regional responses to endothelin in hindquarters vascular bed of cats. Am. J. Physiol. 1989;256:H598-H602). Elucidation of the precise role of ET in essential hypertension must awake the development of sensitive and specific antagonist for ET.

Koyama et al. recently reported the marked elevation in the plasma ET-1-LI level in patients with uremia (Koyama, H., Tabata, T., Nishizawa, Y., Inoue, T., Morii, H., Yamaji, T.: Plasma endothelin levels in patients with uraemia. Lancet 1989;8645:991-992). In the present invention, there was no significant correlation between the plasma ET-1-LI level and serum creatinine level in hypertensive patients with normal renal functions. However,a positive correlation was observed between the plasma ET-1-LI level and the serum creatinine level in a larger population of hypertensives whose serum creatinine level ranged from 0.5 mg/dl to 9.0 mg/dl.

Recently, Suzuki et al. reported the plasma ET-1 level in normal subjects is 1.5 ± 0.32 pg/ml, using the enzyme immunoassay (EIA) (Suzuki, N., Matsumoto, H., Kitada, C., Masaki, T., Fujino, M.: A sensitive sandwich-enzyme immunoassay for human endothelin. J. Immunol. Method 1989;118:245-250), and this value appears to be lower than that in the present invention (Mukoyama, M., Nakao, K., Yamada, T., Itoh, H., Sugawara, A., Saito, Y., Arai, H., Hosoda, K., Shirakami, G., Morii, N., Shiono, S., Imura, H.: A monoclonal antibody against N-terminus of α-atrial natriuretic polypeptide (α-ANP): a useful tool for preferential detection of naturally circulating ANP. Biochem. Biophys. Res. Commun. 1988;151:1277-1284). The difference depends on the specificity of the assay system used in each laboratory. As mentioned above, ET-1-LI in plasma consists of ET-1, big ET and another peptide (6K) and the ratio of ET-1 to total ET-1-LI in plasma is about 1:5 (Saito, Y., Nakao, K., Itoh, H., Yamada, T., Mukoyama, M., Arai, H., Hosoda, K., Shirakami, G., Suga, S., Jougasaki, M., Morichika, S., Imura, H.: Endothelin in human plasma and culture medium of aortic endothelial cells __ Detection and characterization with radioimmunoassay using monoclonal antibody. Biochem. Biophys. Res. Commun. 1989;161;320-326). KY-ET-1-I equally recognizes ET-1 and big ET, and it also recognizes the 6K peptide (Saito, Y., Nakao, K., Itoh, H., Yamada, T., Mukoyama, M., Arai, H., Hosoda, K., Shirakami, G., Suga, S., Jougasaki, M., Morichika, S., Imura, H.: Endothelin in human plasma and culture medium of aortic endothelial cells __ Detection and characterization with radioimmunoassay using monoclonal antibody. Biochem. Biophys. Res. Commun. 1989;161;320-326), though the EIA developed by Suzuki et al. recognizes only ET-1. Therefore, the plasma ET-1 level in normal subjects in the assay system according to the present invention is comparable to that reported by Suzuki et al. However, Ando et al. reported much lower normal value of plasma ET-1-LI level by the RIA (Ando, K., Hirata, Y., Shichiri, M., Emori, T., Marumo, F.: Presence of immunoreactive endothelin in human plasma. FEBS Lett. 1989;245:164-166). The reason responsible for the difference is not clear at present.

For the neutralization experiment, high-affinity antibodies are required. Dissociation constant of ET receptors in vascular smooth muscle cells was reported to be 4 X $10^{10}$ M (Ka = 2.5 X $10^9$ M$^{-1}$) (Hirata, Y.,

Yoshimi, H., Takata, S., Watanabe, T., Kumagai, S., Nakajima, K., Sakakibara, S.: Cellular mechanism of action by a novel vasoconstrictor endothelin in cultured rat vascular smooth muscle cells. Biochem. Biophys. Res. Commun. 1988;154;868-875). Association constants of antibodies developed in the present invention are more than 5 X $10^{10}M^{-1}$. In fact, the purified monoclonal antibody (Monoclonal antibody KY-ET-1-I) attenuated the ET-1-induced vasoconstriction in vitro and pressor action in vivo in the present invention. These observations suggest the possibility that these monoclonal antibodies are useful to investigate roles of endogenous ET in circulatory diseases including essential hypertension.

## Claims

1. A monoclonal antibody against endothelin (ET) having an association constant Ka of not less than 2.1 x $10^{11}M^{-1}$, which is produced by a cell line having the characteristics of hybridoma KY-ET-1-II (FERM BP-2901), hybridoma KY-ET-1-III (FERM BP-2902) or hybridoma KY-ET-1-IV (FERM BP-2903), or a cell line derived therefrom.

2. The monoclonal antibody of Claim 1, wherein a cross-reactivity with a carboxy terminal fragment of ET (ET[17-21]), is less than 0.01 %.

3. A hybridoma producing the monoclonal antibody of Claim 1 or 2.

4. A radioimmunoassay for endothelin, which is characterized by using the monoclonal antibody of Claim 1 or 2.

5. A process for the preparation of a monoclonal antibody according to claim 1 or 2 which comprises culturing a cell line having the characteristics of hybridoma KY-ET-1-II (FERM BP-2901), hybridoma KY-ET-1-III (FERM BP-2902) or hybridoma KY-ET-1-IV (FERM BP-2903), or a cell line derived therefrom or injecting the cell line into mice to produce ascitic fluid, and isolating the monoclonal antibody therefrom.

## Patentansprüche

1. Monoclonaler Antikörper gegen Endothelin (ET) mit einer Assoziationskonstante $K_a$ von mindestens 2,1 x $10^{11}$ $M^{-1}$, der von einer Zellinie mit den Merkmalen des Hybridoms KY-ET-1-II (FERM BP-2901), des Hybridoms KY-ET-1-III (FERM BP-2902), des Hybridoms KY-ET-1-IV (FERM BP-2903) oder einer davon abstammenden Zellinie produziert wird.

2. Monoclonaler Antikörper nach Anspruch 1, wobei die Kreuzreaktivität mit dem Carboxy-terminalen Fragment von ET (ET[17-21]) kleiner als 0,01 % ist.

3. Hybridom, das den monoclonalen Antikörper nach Anspruch 1 oder 2 produziert.

4. Radioimmuntest für Endothelin, der durch die Verwendung des monoclonalen Antikörpers nach Anspruch 1 oder 2 gekennzeichnet ist.

5. Verfahren zur Herstellung eines monoclonalen Antikörpers nach Anspruch 1 oder 2, umfassend die Züchtung einer Zellinie mit den Merkmalen des Hybridoms KY-ET-1-II (FERM BP-2901), des Hybridoms KY-ET-1-III (FERM BP-2902) oder des Hybridoms KY-ET-1-IV (FERM BP-2903) oder einer davon abstammenden Zellinie oder Injektion der Zellinie in Mäuse zur Produktion von Ascitesflüssigkeit und Isolierung des monoclonalen Antikörpers daraus.

## Revendications

1. Anticorps monoclonal contre l'endothéline (ET) ayant une constante d'association $K_a$ de pas moins de 2,1 x $10^{11}M^{-1}$, qui est produit par une lignée cellulaire ayant les caractéristiques de l'hybridome KY-ET-1-II (FERM BP-2901), de l'hybridome KY-ET-1-III (FERM BP-2902) ou de l'hybridome KY-ET-1-IV (FERM BP-2903) ou par une lignée cellulaire provenant de celle-ci.

2. Anticorps monoclonal suivant la revendication 1, dans lequel une réactivité croisée avec un fragment terminal carboxy de ET (ET[17-21]), est inférieure à 0,01 %.

**3.** Hybridome produisant l'anticorps monoclonal de la revendication 1 ou 2.

**4.** Radioimmunoessai pour l'endothéline, qui est caractérisé par l'utilisation de l'anticorps monoclonal de la revendication 1 ou 2.

**5.** Procédé de préparation d'un anticorps monoclonal suivant l'une ou l'autre des revendications 1 et 2, qui comprend la culture d'une lignée cellulaire ayant les caractéristiques de l'hybridome KY-ET-1-II (FERM BP-2901), de l'hybridome KY-ET-1-III (FERM BP-2902) ou de l'hybridome KY-ET-1-IV (FERM BP-2903), ou d'une lignée cellulaire dérivant de celle-ci ou l'injection de la lignée cellulaire chez des souris pour produire un fluide ascitique, et l'isolement de l'anticorps monoclonal.

# F I G . 1

FIG.2

FIG.3

17

FIG. 4

FIG. 5

# FIG. 6

# FIG. 7

## F I G.8

## F I G.9

< 30pg/ml                    ≧ 30pg/ml

17%                          63%

△ WHO   Stage I
◿ WHO   Stages II & III

FIG. 10

FIG. 11